# EUROPEAN PATENT APPLICATION

(11) **EP 2 389 953 A1**
(43) Date of publication of application: **30.11.2011**
(21) Application number: 11004531.7
(22) Date of filing: 09.06.2004
(51) Int. Cl.: A61K 39/385, A61K 39/395, A61K 45/06, C07K 16/28

(54) **Method of inhibiting receptor tyrosine kinases with an extracellular antagonist and an intracellular antagonist**

(30) Priority: 09.06.2003 US 477796 P
(62) Divisional of application: 04754904.3
(71) Applicant: Waksal, Samuel, Minersville, PA 17954-0670 (US)
(72) Inventor: Waksal, Samuel, Minersville, PA 17954-0670 (US)
(74) Representative: Grünecker, Kinkeldey, Stockmair & Schwanhäusser

(57) **Abstract**

The present invention relates to methods of inhibiting receptor tyrosine kinases by utilizing a combination of both an extracellular and an intracellular RTK antagonist. The extracellular RTK antagonist is a biological molecule or a small molecule that inhibits activation of the receptor tyrosine kinase by interacting with the extracellular binding region of the receptor. The intracellular RTK antagonist is a biological molecule or small molecule that inhibits tyrosine kinase activity of the receptor tyrosine kinase by interacting with the receptor's intracellular region bearing a kinase domain or by interacting with an intracellular protein involved in the signaling pathway of the receptor tyrosine kinase. The present invention also provides methods of treating tyrosine kinase-dependent diseases, and compositions for use in such methods thereof, by administering a combination of both an extracellular and an intracellular RTK antagonist.

## Description

### FIELD OF THE INVENTION

The present invention relates to methods of inhibiting receptor tyrosine kinases (RTKs) with an extracellular RTK antagonist and an intracellular RTK antagonist. In particular, the present invention relates to methods of treating tyrosine kinase-dependent diseases and conditions in mammals by administering both the extracellular and intracellular RTK antagonists.

### BACKGROUND OF THE INVENTION

RTKs are transmembrane proteins that have been implicated in the control and regulation of several cellular processes such as cell proliferation and differentiation, promotion of cell survival, and modulation of cellular metabolism. Ligands for RTKs are soluble or membrane-bound peptides or protein hormones. Generally, binding of a ligand to the RTK stimulates the receptor's tyrosine kinase activity, which subsequently stimulates a signal-transduction cascade of biochemical and physiologic changes, culminating in DNA synthesis and cell division. Examples of such receptors includes epidermal growth factor receptor (EGFR), insulin receptor, platelet-derived growth factor receptor (PDGFR), vascular endothelial growth factor receptor (VEGFR), fibroblast growth factor receptor (FGFR), hepatocyte growth factor receptor (HGFR), and nerve growth factor receptor (NGFR).

Generally, RTKs have an extracellular region, a transmembrane hydrophobic domain, and an intracellular region bearing a kinase domain. When a ligand binds to the extracellular binding region on the cell surface of such an RTK, a conformational change in the receptor is generated, which exposes the phosphorylation sites of the intracellular tyrosine kinase domains. A conformation change in the receptor can also be generated following homo or heterodimerization with a related RTK. Phosphorylation of these proliferation and differentiation.

In addition, binding of a ligand causes many RTKs to dimerize and the protein kinase of each receptor monomer then phosphorylates a distinct set of tyrosine residues in the intracellular region of its dimer partner, a process referred to as autophosphorylation. Autophosphorylation generally occurs in two stages. First, tyrosine residues in the phosphorylation lip near the catalytic site are phosphorylated. This leads to a conformational change that facilitates binding of ATP or protein substrates to the receptor.

The phosphorylated receptor then serves as a docking site for other proteins involved in the RTK-mediated signal transduction. These proteins include the adapter protein GRB2, which binds to a specific phosphotyrosine on the activated RTK and binds to Sos, another intracellular protein, which is turn interacts with an inactive Ras-GDP complex (Ras is a GTP-binding switch protein that alternates between an active "on" state with a bound GTP and an inactive "off' state with a bound GDP). The guanine nucleotide-exchange factor (GEF) activity of Sos then promotes formation of the active Ras-GTP complex. Ras then induces a kinase cascade that culminates in activation of MAP kinase. In particular, activated Ras binds to the N-terminal domain of Raf, a serine-threonine kinase. Raf, in turn, binds to and phosphorylates MEY, a dual-specificity protein kinase that phosphorylates both tyrosine and serine residues and that activates MAP kinase,another serine-threonine kinase. MAP kinase phosphorylates many different proteins that mediate cellular responses, including nuclear transcription factors.

Aberrations in the signaling pathways associated with RTKs are thought to contribute to a number of pathological outcomes including cancer, cardiovascular disease, inflammatory disease, and other pròliferative diseases. For example, some RTKs have been identified in studies on human cancers associated with mutant forms of growth-factor receptors, which sends a proliferative signal to cells even in the absence of growth factor. One such mutant receptor, encoded at the *neu* locus, is thought to contribute to the uncontrolled proliferation of certain human breast cancers. Specific members of RTKs have also been implicated in various human cancers.

One RTK involved in tumorigenesis is the EGF receptor family, which includes the EGF receptor (EGFR, also known as erbB-1/HER1), HER2 (also known as c-neu/erbB-2), erbB-3/HER3, and erbB-4/HER4. For example, EGFR and HER2 are thought to play a critical role in processes that regulate tumor cell growth and survival. In particular, EGFR has been implicated in several pathways that affect survival and protection from apoptosis, dedifferentiation, metastasis (including cell migration and invasion) and EGFR has also been implicated in angiogenesis, the ability of solid tumors to create their own vascular system by forming new blood vessels.

It has been reported that many human tumors express or over-express one or more members of the EGF family of receptor. Specifically, EGFR presence seems to correlate with poor prognosis, increased risk of tumor spreading, and shorter overall survival in certain tumor types. It is also thought that the poor overall response to standard chemotherapy and radiation in late-stage disease may be due to the ability of EGFR to repair damage in tumor cells that are not killed by such standard approaches. In addition, research has shown that HER2 positive metastatic breast cancer is an especially aggressive disease, resulting in a greater likelihood of recurrence, poorer prognosis and approximately half the life expectancy as compared with HER2 negative breast cancer. HER2 protein overexpression is observed in 25-30% of primary breast cancers.

Members of the VEGFR family have also been implicated in tumorigenesis. For example, these receptors are thought to play a role in tumor formation, angiogenesis and tumor growth. VEGFRs are selectively expressed on endothelial cells during, for example, embryogenesis and tumor formation and VEGFR antagonists have been developed that block signaling by VEGF receptors expressed on endothelial cells to reduce tumor growth. VEGF receptors have also been found on some non-endothelial cells, such as tumor cells producing VEGF, wherein an endothelial-independent autocrine loop is generated to support tumor growth.

Accordingly, by developing appropriate inhibitors, regulators, or modulators of RTKs, the signaling pathways of RTKs may be modulated to treat or prevent these pathological outcomes. Because of the involvement of EGFR and VEGFR in tumorigenesis, these RTKs have been specifically targeted for anti-cancer drug therapy. This therapy has predominantly included either a monoclonal antibody that blocks binding of a ligand to the extracellular domain of the receptor or a synthetic tyrosine kinase inhibitor that acts directly on the intracellular region of the RTK to prevent signal transduction.

There are various monoclonal antibody inhibitors currently in clinical trials. One such example is cetuximab, which is a chimeric (human/mouse) monoclonal antibody that blocks ligand binding to EGFR, prevents receptor activation, and inhibits growth of cells in culture. Another example is ABX-EGF, which is a fully human monoclonal antibody specific to EGFR that reportedly blocks binding of EGF and TFG-α. Herceptin® (trastuzumab) is a humanized antibody approved for the treatment of HER2 positive metastatic breast cancer, which is designed to target and block the function of HER2 protein overexpression.

In addition, clinical trials are currently being conducted on various small molecule inhibitors. An example of a tyrosine kinase inhibitor is Iressa™, which is a small molecule epidermal growth factor receptor tyrosine kinase inhibitor that reportedly inhibits EGFR tyrosine kinase activity, is cytostatic towards a range of human cancer cells that express functional EGFR, and can inhibit tumor cell proliferation via up-regulation of p27.

Although current small molecule therapeutics that target RTKs have been found to suppress growth of susceptible tumors for as long as dosing continues, they are associated with at times severe side effects. It has been reported that once dosing with the small molecule is terminated, tumor regrowth occurs, which can occur at an even greater rate than prior to treatment. Furthermore, continuous dosing of small molecule tyrosine kinase inhibitors has been shown to result in other side effects such as rash, diarrhea, mucositis, and neutropenia.

### SUMMARY OF THE INVENTION

The present invention provides a method of inhibiting receptor tyrosine kinases (RTKs) by using an extracellular RTK antagonist and an intracellular RTK antagonists. In particular, the present invention provides a method of treating tyrosine kinase-dependent diseases and conditions, such as tumor growth, in mammals by administering both the extracellular and intracellular RTK antagonists. Such treatment results in an enhanced or synergistic effect on tumor growth inhibition compared to administration of either solely an extracellular RTK antagonist or solely an intracellular RTK antagonist. The present invention also provides pharmaceutical compositions comprising an extracellular RTK antagonist and an intracellular RTK antagonist.

### DETAILED DESCRIPTION OF THE INVENTION

The present invention provides a method of inhibiting RTKs with an extracellular RTK antagonist and an intracellular RTK antagonist. An RTK is a transmembrane, cell-surface receptor having an extracellular region, a transmembrane hydrophobic domain, and an intracellular region bearing a kinase domain. Following activation of the extracellular region, which can occur through ligand binding or homo or heterodimerization with another RTK, the intracellular kinase domain is activated. An RTK signal transduction pathway is initiated when the intracellular domain is activated and tyrosine kinase activity stimulated, thereby activating various genes, initiating cell cycle progression and, ultimately, cellular proliferation and differentiation.

Preferably, the RTK is a member of the EGFR family such as EGFR or erbB-1, erbB-2, erbB-3, or erbB-4. More preferably, the RTK is EGFR, which is a 170 kDa membrane-spanning glycoprotein that binds to, for example, EGF, TNF-α, amphiregulin, heparin-binding EGF (HB-EGF), betacellulin, epiregulin, and NRG2-α. Also preferably, the RTK is HER2, a proto-oncogene that encodes a transmembrane receptor protein of 185 kDa. The RTK may also be a member of the VEGF receptor (VEGFR) family, which includes VEGFR-1, VEGFR-2, VEGFR-3, neuropilin-1 and neuropilin-2. Ligands that bind to VEGFR-1 and VEGFR-2 include isoforms of VEGF (VEGF₁₂₁, VEGF₁₄₅, VEGF₁₆₅, VEGF₁₈₉ and VEGF₂₀₆).

Non-limiting examples of other RTKs to which an antagonist according to the present invention can bind include members of the PDGF receptor (PDGFR) family such as PDGFR-α (which binds to PDGF-AA, PDGF-BB, and PDGF-AB) and PDGFR-β (which binds to PDGF-BB); members of the FGF receptor (FGFR) family such as FGRF-1 and FGFR-2; members of the HGF receptor (HGFR) family; members of the NGR receptor (NGFR) family such as CD27 and CD40; and members of the insulin receptor family such as insulin receptor (IR), type 1 insulin-like growth factor I receptor (IGF-IR) and insulin receptor-related receptor (IRR).

The extracellular RTK antagonists, in the context of the present invention, interact with the extracellular binding region of the RTK through sufficient physical or chemical interaction between the RTK antagonist and the extracellular binding region of the receptor, such that tyrosine kinase activity is inhibited. One of skill in the art would appreciate that examples of such chemical interactions, which include association or bonding, are known in the art and include covalent bonding, ionic bonding, hydrogen bonding, and the like between the RTK antagonist and the extracellular binding region.

The intracellular RTK antagonists, in the context of the present invention, inhibit the tyrosine kinase activity of the RTK by preventing receptor phosphorylation and/or the phosphorylation of other proteins involved in the various RTK signaling pathways. The intracellular RTK antagonist may inhibit the tyrosine kinase activity of the RTK by binding to or inhibiting activation of the intracellular region bearing a kinase domain or by binding to or inhibiting activation of any intracellular protein involved in the signaling pathway of the RTK.

It should be appreciated, of course, that while both the extracellular antagonist and the intracellular antagonist should function to inhibit the same RTK pathway, these pathways can be distinct signaling pathways. Therefore, the pathways may function completely independently of each other, and the extracellular pathway may be activated when the intracellular pathway is not and vise-a-versa. Moreover, the mechanism of action of each pathway may be different; thus also resulting is different activation and signaling.

Although not wishing to be bound by theory, it is thought that the extracellular RTK antagonist inhibits all signal transduction cascades initiated by the conformation changes in the extracellular region of the RTK following RTK activation. This inhibition includes surface RTKs as well as those RTKs that have been internalized within a cell. For example, it is thought that activated RTKs can be internalized via a clatherin-coated pit into an endosome, while still maintaining their signaling activity. Following internalization, such receptors are either recycled back to the cell surface or degraded in the endosome or lysosome. Binding of a ligand to the receptor may promote recycling of the receptor, while binding of either another receptor (i.e., a homo or heterodimer) or an antagonist to the receptor may promote degradation of the RTK.

The extracellular and intracellular RTK antagonists, in the context of the present invention, can be biological molecules, small molecules, or any other substance that inhibits activation of an RTK by interaction with the extracellular binding region of the receptor (i.e., extracellular antagonist) or inhibits phosphorylation by interaction with the intracellular tyrosine kinase domain or any other intracellular protein involved in the pathway (i.e., intracellular antagonist), thereby ultimately inhibiting gene activation or cellular proliferation. Generally, the RTK antagonists decrease the activation of an RTK, without necessarily completely preventing or stopping activation of the RTK.

Biological molecules, in the context of the present invention, include all amino acids, nucleotides, lipids and polymers of monosaccharides that generally have a molecular weight greater than 650 D. Thus, biological molecules include, for example, oligopeptides, polypeptides, peptides, and proteins, oligonucleotides and polynucleotides such as, for example, DNA and RNA, and oligosaccharides and polysaccharides. Biological molecules further include derivatives of any of the molecules described above. For example, derivatives of biological molecules include lipids and glycosylation derivatives or oligopeptides, polypeptides, peptides, and proteins. Derivatives of biological molecules further include lipid derivatives of oligosaccharides and polysaccharides, e.g. lipopolysaccharides. Most typically, biological molecules are antibodies or functional derivatives thereof.

Such antibodies according to the present invention may be, for example, naturally-occurring antibodies, bivalent fragments such as (Fab')₂, monovalent fragments such as Fab, single chain antibodies such as single chain Fvs (scFv), single domain antibodies, multivalent single chain antibodies, diabodies, triabodies, and the like, which may be mono or bi-specific, that bind specifically with antigens. The antibodies according to the present invention may also be single domain antibodies, which bind efficiently and include a single antibody variable domain that provides efficient binding. Antibodies that are homodimers of heavy chains and are devoid of light chains and the first constant domain may also be used.

In general, the antibodies of the present invention comprise human V_{H} and V_{L} framework regions (FWs) as well as human complementary determining regions (CDRs). Preferably, the entire V_{H} and V_{L} variable domains are human or derived from human sequences. Also, the variable domains of the antibodies of the present invention may be a complete antibody heavy or light chain variable domain, or it may be a functional equivalent or a mutant or derivative of a naturally occurring domain, or a synthetic domain constructed using techniques known to those skilled in the art. For instance, it is possible to join together domains corresponding to antibody variable domains that are missing at least one amino acid. The important characterizing feature is the ability of each domain to associate with a complementary domain to form an antigen-binding site.

V_{L} and V_{H} domains from a selected source may be incorporated into chimeric antibodies with functional human constant domains. Antibodies of the invention can also be "humanized," and comprise one or more complementarity determining regions (CDRs) of non-human origin grafted to human framework regions (FRs). Alternatively, human I binding domains or antibodies can be obtained from transgenic animals, into which unrearranged human Ig gene segments have been introduced and in which the endogenous mouse Ig genes have been inactivated (reviewed in Brüggemann and Taussig (1997) Curr. Opin. Biotechnol. 8, 455-458). Monoclonal antibodies, produced from such mice are human.

Functional equivalents of antibodies are also contemplated by the present invention and include polypeptides with amino acid sequences substantially the same as the amino acid sequence of the variable or hypervariable regions of the full length antibodies. "Substantially the same" amino acid sequence is defined herein as a sequence with at least 70%, preferably at least about 80%, and more preferably at least about 90% homology to another amino acid sequence, as determined by the FASTA search method in accordance with Pearson and Lipman, Proc. Natl. Acad. Sci. USA 85, 2444-8 (1988). Antibodies of the present invention also include those for which binding characteristics (e.g., affinity and specificity) have been improved by direct mutation, methods of affinity maturation, phage display, or chain shuffling.

An antibody or mixture of antibodies is preferably employed as the extracellular RTK antagonist. The antibody binds to the extracellular domain and preferably neutralizes RTK activation, for example by blocking receptor dimerization and/or ligand binding. More preferably the extracellular RTK antagonist is an EGFR antibody.

One example of such an EGFR antibody is cetuximab (IMC-C225), which is a chimeric (human/mouse) IgG monoclonal antibody. *See e.g.,* U.S. Patent No. 4,943,533 (Mendelsohn et al.*);* U.S. Patent No. 6,217,866 (Schlessinger et al.*);* U.S. Application Nos. 08/973,065 (Goldstein et al.*)* and 09/635,974 (Teufel); WO 99/60023 (Waksal et al.*)* and WO 00/69459. Cetuximab specifically binds to EGFR and blocks binding of a ligand, such as EGF. This blockade interferes with the effects of EGFR activation and results in inhibition of tumor growth, tumor invasion, metastases, cell repair and angiogenesis. In addition, or alternatively, cetuximab may promote internalization of the receptor-antibody complex, preventing further stimulation of the receptor by its ligand or by any other mechanism.

Another example of an EGFR antibody is ABX-EGF, which is a fully human IgG₂ monoclonal antibody specific for EGFR. ABX-EGF binds EGFR with high specificity, blocking binding of EGFR to both of its ligands, EGF and TGF-α. *See e.g.,* Figlin et al., Abstract 1102 presented at the 37th Annual Meeting of ASCO, San Francisco, CA, 12-15 May 2001. The sequence and characterization of ABX-EGF, which was formerly known as clone E7.6.3, is disclosed in U.S. Patent No. 6,235,883 (Abgenix, Inc.) at col. 28, line 62 through col. 29, line 36 and in Fig. 29-34. *See* Yang et al., Critical Rev. Oncol./Hematol., 38(1): 17-23, 2001.

Herceptin® (trastuzumab) is a recombinant DNA-derived humanized monoclonal antibody that selectively binds with high affinity in a cell-based assay (Kd of 5 nM) to the extracellular domain of the human EGFR2 protein, HER2. The antibody is an IgG₁ kappa that contains human framework regions with the complementarity-determining regions of a murine antibody (4D5) that binds to HER2. *See, e.g.,* International Patent Publication No. WO 01/89566 (Mass).

Other EGFR antibodies that can be used as the extracellular RTK according to the present invention include EMD 72000 (Merck KGaA), which is a humanized version of the murine anti-EGFR monoclonal antibody EMD 55900; h-R3 (TheraCIM), which is a humanized anti-EGFR monoclonal antibody; Y10, which is a murine monoclonal antibody and was raised against a murine homologue of the human EGFRvIII mutation; and MDX-447 (Medarex). *See* U.S. Patent Nos. 5,558,864 (Bendig et al.), 5,884,093 (Kettleborough et al.), 5,891,996 (Mateo de Acosta del Rio et al.).

The extracellular RTK antagonist according to the present invention may also be a VEGFR antibody. Cell lines that produce VEGFR antibodies include the DC101 hybridoma cell line that produces rat anti-mouse VEGFR-2 monoclonal antibody (ATCC HB 11534); the M25.18A1 hybridoma cell line that produces mouse anti-mouse VEGFR-2 monoclonal antibody MAb 25 (ATCC HB 12152); the M73.24 hybridoma cell line that produces mouse anti-mouse VEGFR-2 monoclonal antibody MAb 73 (ATCC HB 12153); and the cell line that produces MAb 6.12 that binds to soluble and cell surface-expressed VEGFR-1 (ATCC PTA-3344). Other hybridomas that produce anti-VEGFR-1 antibodies include, but are not limited to, hybridomas KM1730 (deposited as FERM BP-5697); KM1731 (deposited as FERM BP-5718); KM1732 (deposited as FERM BP-5698); KM1748 (deposited as FERM BP-5699); and KM1750 (deposited as FERM BP-5700) disclosed in WO 98/22616, WO 99/59636, Australian accepted application no. AU 1998 50666 B2, and Canadian application no. CA 2328893. Further examples of VEGFR-2 specific antibodies include IMC-1C11 *(see* WO 00/44777 (Zhu et al.*);* WO 01/90192 (Zhu)) and IMC-2C6 *(see Lu et al.,* 2002; PCT/US02/20332 (Zhu)).

Other VEGFR antagonists are known in the art. Some examples of VEGFR antagonists are described in U.S. Application Nos. 07/813,593; 07/906,397; 07/946,507; 07/977,451; 08/055,269; 08/252,517; 08/601,891; 09/021,324; 09/208,786; and 09/919,408 (all to Lemischka *et al.);* U.S. Patent No. 5,840,301 (Rockwell et al.*);* U.S. Application Nos. 08/706,804; 08/866,969; 08/967,113; 09/047,807; 09/401,163; and 09/798,689 (all to Rockwell *et al.);* U.S. Application No. 09/540,770 (Witte et al.*);* and PCT/US01/06966 (Liao et al.*).* U.S. Patent No. 5,861,301 (Terman et al.*),* Terman et al. Oncogene 6: 1677-1683 (September 1991), WO 94/10202 (Ferrara et al.*),* and WO 95/21865 (Ludwig) disclose VEGFR antagonists and, specifically, anti-VEGFR-2 antibodies. In addition, PCT/US95/01678 (Kyowa Hakko) describes anti-VEFGR-2 antibodies. Anti-VEGFR antibodies are also described in U.S. Application No. 09/976,787 (Zhu et al.*).* U.S. Patent Nos. 6,177,401 (Ullrich et al.*),* 5,712,395 (App et al.*),* and 5,981,569 (App et al.*)* describe VEGFR antagonists that are organic molecules. In addition, bi-specific antibodies (BsAbs), which are antibodies that have two different antigen-binding specificities or sites, directed to KDR and VEGFR-1 are known. *See, e.g.,* U.S. Application No. 09/865,198 (Zhu); 60/301,299 (Zhu).

One specific VEGF antagonist is Avastin™ (bevacizumab, Genentech), a recombinant, humanized monoclonal antibody to VEGF (rhuMAb-VEGF). Avastin, which is designed to bind to and inhibit VEGF, is involved in a Phase III clinical study in metastatic colorectal cancer patients with a primary endpoint of improving overall survival.

The intracellular RTK antagonists are preferably small molecules. Some examples of small molecules include organic compounds, organometallic compounds, salts of organic compounds and organometallic compounds, and inorganic compounds. Atoms in a small molecule are linked together via covalent and ionic bonds; the former is typical for small organic compounds such as small molecule tyrosine kinase inhibitors and the latter is typical of small inorganic compounds. The arrangement of atoms in a small organic molecule may represent a chain, *e.g.* a carbon-carbon chain or carbon-heteroatom chain or may represent a ring containing carbon atoms, *e.g.* benzene or a policyclic system, or a combination of carbon and heteroatoms, *i.e.,* heterocycles such as a pyrimidine or quinazoline. Although small molecules can have any molecular weight they generally include molecules that would otherwise be considered biological molecules, except their molecular weight is not greater than 650 D. Small molecules include both compounds found in nature, such as hormones, neurotransmitters, nucleotides, amino acids, sugars, lipids, and their derivatives as well as compounds made synthetically, either by traditional organic synthesis, bio-mediated synthesis, or a combination thereof. *See e.g.* Ganesan, Drug Discov. Today 7(1): 47-55 (Jan. 2002); Lou, Drug Discov. Today, 6(24): 1288-1294 (Dec. 2001).

More preferably, the small molecule to be used as an intracellular RTK antagonist according to the present invention is an intracellular EGFR antagonist that competes with ATP for binding to EGFR's intracellular binding region having a kinase domain or to proteins involved in the signal transduction pathways of EGFR activation. Examples of such signal transduction pathways include the ras-mitogen activated protein kinase (MAPK) pathway, the phosphatidylinosital-3 kinase (P13K)-Akt pathway, the stress-activated protein kinase (SAPK) pathway, and the signal transducers and activators of transcription (STAT) pathways. Non-limiting examples ofproteins involved in such pathways (and to which a small molecule EGFR antagonist according to the present invention can bind) include GRB-2, SOS, Ras, Raf, MEK, MAPK, and matrix metalloproteinases (MMPs).

One example of a small molecule EGFR antagonist is IRESSA™ (ZD1939), which is a quinozaline derivative that functions as an ATP-mimetic to inhibit EGFR. *See* U.S. Patent No. 5,616,582 (Zeneca Limited); WO 96/33980 (Zeneca Limited) at p. 4; *see also,* Rowinsky et al., Abstract 5 presented at the 37th Annual Meeting of ASCO, San Francisco, CA,12-15 May 2001; Anido et al., Abstract 1712 presented at the 37th Annual Meeting of ASCO, San Francisco, CA, 12-15 May 2001.

Another examples of a small molecule EGFR antagonist is TARCEVA™ (OSI-774), which is a 4-(substitutedphenylamino)quinozaline derivative [6,7-Bis(2-methoxy-ethoxy)-quinazolin-4-yl]- (3-ethynyl-phenyl)amine hydrochloride] EGFR inhibitor. *See* WO 96/30347 (Pfizer Inc.) at, for example, page 2, line 12 through page 4, line 34 and page 19, lines 14-17. *See also* Moyer et al., Cancer Res., 57: 4838-48 (1997); Pollack et al., J. Pharmacol., 291: 739-48 (1999). TARCEVA™ may function by inhibiting phosphorylation of EGFR and its downstream PI3/Akt and MAP (mitogen activated protein) kinase signal transduction pathways resulting in p27-mediated cell-cycle arrest. *See* Hidalgo et al., Abstract 281 presented at the 37th Annual Meeting of ASCO, San Francisco, CA, 12-15 May 2001.

Other small molecules are also reported to inhibit EGFR, many of which are thought to bind to the tyrosine kinase domain of an EGFR. These include tricyclic compounds such as the compounds described in U.S. Patent No. 5,679,683; quinazoline derivatives such as the derivatives described in U.S. Patent No. 5,616,582; and indole compounds such as the compounds described in U.S. Patent No. 5,196,446. Examples of such small molecule EGFR antagonists are described in WO 91/116051, WO 96/30347, WO 96/33980, WO 97/27199 (Zeneca Limited). WO 97/30034 (Zeneca Limited), WO 97/42187 (Zeneca Limited), WO 97/49688 (Pfizer Inc.), WO 98/33798 (Warner Lambert Company), WO 00/18761 (American Cyanamid Company), and WO 00/31048 (Warner Lambert Company). Naturally derived EGFR tyrosine kinase inhibitors include genistein, herbimycin A, quercetin, and erbstatin.

Examples of specific small molecule EGFR antagonists include Cl-1033 (Pfizer), which is a quinozaline (N-[4-(3-chloro-4-fluoro-phenylamino)-7-(3-morpholin-4-yl-propoxy)-quinazolin-6-yl]-acrylamide) inhibitor of tyrosine kinases, particularly EGFR and is described in WO 00/31048 at page 8, lines 22-6; PKI166 (Novartis), which is a pyrrolopyrimidine inhibitor of EGFR and is described in WO 97/27199 at pages 10-12; GW2016 (GlaxoSmithKline), which is an inhibitor of EGFR and HER2; EKB569 (Wyeth), which is reported to inhibit the growth of tumor cells that overexpress EGFR or HER2 *in vitro* and *in vivo;* AG-1478 (Tryphostin), which is a quinazoline small molecule that inhibits signaling from both EGFR and erbB-2; AG-1478 (Sugen, Pharmacia and Repligen), which is bisubstrate inhibitor that also inhibits protein kinase CK2; PD 153035 (Parke-Davis) which is reported to inhibit EGFR kinase activity and tumor growth, induce apoptosis in cells in culture, and enhance the cytotoxicity of chemotherapeutic agents; SPM-924 (Schwarz Pharma), which is a tyrosine kinase inhibitor targeted for treatment of prostrate cancer; CP-546,989 (OSI Pharmaceuticals), which is reportedly an inhibitor of angiogenesis for treatment of solid tumors; ADL-681, which is a EGFR kinase inhibitor targeted for treatment of cancer; PD 158780, which is a pyridopyrimidine that is reported to inhibit the tumor growth rate of A4431 xenografts in mice; CP-358,774, which is a quinzoline that is reported to inhibit autophosphorylation in HN5 xenografts in mice; ZD1839, which is a quinzoline that is reported to have antitumor activity in mouse xenograft models including vulvar, NSCLC, prostrate, ovarian, and colorectal cancers; CGP 59326A, which is a pyrrolopyrimidine that is reported to inhibit growth of EGFR-positive xenografts in mice; PD 165557 (Pfizer); CGP54211 and CGP53353 (Novartis), which are dianilnophthalimides.

The intracellular RTK antagonist can also be an inhibitor of the ras protein, a protein involved in the signal transduction pathway of EGFR. Such inhibitors can target farnesyltransferase, which is an enzyme that activates the ras protein and such inhibitors include, for example, R115777 Zamestra (Ortho-Biotech), which is used in combination with gemcitabine for treatment of ras-dependent tumors; SCH66336 (Schering Plough), which is reported for treatment of a variety of solid tumors, including metastatic bladder cancer, advanced pancreatic cancer, and head and neck squamous cell carcinoma; BMS-214662 Ptase (Bristol-Myers Squibb), which is reported for treatment for acute leukemia, myelodysplastic syndrome and chronic myeloid leukemia; L-778,123 (Merck), which is a peptidomimetic farnesyl protein transferase (FPTase) inhibitor reported for treatment of recurrent or refractory solid tumors; CP-609-754 (OSI Pharmaceuticals and Pfizer), which is an inhibitor of ras farnesylation reported for treatment of solid tumor cancers; and AZD-3409 (AstraZeneca), which is a farnesyl protein transferase inhibitor targeted for treatment of solid tumors.

The intracellular RTK antagonist can also be a ras-raf modulator, such as 43-9006 (Onyx Pharmaceuticals/Bayer), which is a small molecule that targets cells with mutations in the ras gene to inhibit raf kinase and block the ras signaling pathway for treatment of colon, lung, pancreatic and other cancers, and other proliferative diseases; ras antagonist FTS (Thyreos), which reportedly inactivates mutant ras proteins for treatment of melanoma, pancreatic, colon, lung, breast and other cancers.

Other examples of intracellular RTK antagonists, which are not necessarily small molecules and/or antagonists specific for only EGFR are styrl-substituted heteroaryl compounds such as the compounds described in U.S. Patent No. 5,656,655; bis mono- and bicyclic aryl and heteroaryl compounds such as the compounds described in U.S. Patent No. 5,646,153; PD 153035 described in Fry et al. (265 Science 1093-1095 (August 1994)); tyrphostins such as those described in Osherov et al. (J. Biol. Chem., Vol. 268, No. 15 pp. 11134-11142 (1993)); and PD166285 (6-aryl-pyriodo[2,3-d] pyrimidines) described in Panek et al. (J. Pharm and Exp. Therapeutics, Vo. 283, No. 3, pp. 1433-1444 (1997)).

The intracellular RTK antagonist can also be a small molecule VEGFR antagonist such as AXD-6474 (AstraZeneca), which is reportedly an angiogenesis inhibitor; CEP-5214, which is a signal transduction modulator; or ZD-6474, which is a inhibitor of VEGFR tyrosine kinase that reportedly disrupts a signaling pathway in angiogenesis for treatment of advanced solid tumors.

The above-mentioned extracellular and intracellular RTK antagonists are only exemplary and other extracellular and intracellular RTK antagonists that inhibit tyrosine kinase activity are well known to one of skill in the art and/or are readily identifiable and therefore are within the scope of the present invention. To identify such other antagonists, a variety of tyrosine kinase inhibition assays well known to one of skill in the art can be performed.

For example, because the antagonists of the present invention generally involve inhibition or regulation of phosphorylation events, phosphorylation assays may be useful in determining antagonists useful in the context of the present invention. Such assays can detect the autophosphorylation level of recombinant kinase receptors, and/or phosphorylation of natural or synthetic substrates. The phosphorylation can be detected, for example, by using an antibody specific for phosphotyrosine in an ELISA assay or a western blot. Such phosphorylation assays to determine tyrosine kinase activity are described in Panek et al., J. Pharmacol. Exp. Thera., 283: 1433-44 (1997) and Batley et al., Life Sci., 62:143-50 (1998). Detailed descriptions of conventional assays, such as those employed in phosphorylation and ELISA assays, can be obtained from numerous publication, including Sambrook, J. et al., (1989) Molecular Cloning: A Laboratory Manual, 2nd ed., Cold Spring Harbor Laboratory Press.

In addition, methods for detection of protein expression can be utilized, wherein the proteins being measured are regulated by tyrosine kinase activity. These methods include immunohistochemistry (IHC) for detection of protein expression, fluorescence in *situ* hybridization (FISH) for detection of gene amplification, competitive radioligand binding assays, solid matrix blotting techniques, such as Northern and Southern blots, reverse transcriptase polymerase chain reaction (RT-PCR) and ELISA. *See, e.g.,* Grandis et al., Cancer, 78:1284-92. (1996); Shimizu et al., Japan J. Cancer Res., 85:567-71 (1994); Sauter et al., Am. J. Path., 148:1047-53 (1996); Collins, Glia, 15:289-96 (1995); Radinsky et al., Clin. Cancer Res., 1:19-31 (1995); Petrides et al., Cancer Res., 50:3934-39 (1990); Hoffmann et al., Anticancer Res., 17:4419-26 (1997); Wikstrand et al., Cancer Res., 55:3140-48 (1995).

*In* vivo assays can also be utilized to detect tyrosine kinase inhibition. For example, receptor tyrosine kinase inhibition can be observed by mitogenic assays using cell lines stimulated with a receptor ligand in the presence and absence of an inhibitor. For example, HUVEC cells stimulated with VEGF can be used to assay VEGFR inhibition. Another method involves testing for inhibition of growth of EGFR- or VEGF-expressing tumor cells, using for example, human tumor cells injected into a mouse. *See* U.S. Patent No. 6,365,157 (Rockwell et al.*).*

In another aspect, the present invention provides methods of treating tyrosine kinase-dependent diseases and conditions in mammals by administering a therapeutically effective amount of an extracellular RTK antagonist and an intracellular RTK antagonist. Treating such conditions and disorders includes reduce the effects of, prevent, inhibit the proliferation of, or alleviate the symptoms of tyrosine kinase dependent diseases. One skilled in the art would easily be able to diagnose such conditions and disorders using known, conventional tests.

Administering the extracellular and intracellular RTK antagonists includes delivering the RTK antagonists to a mammal by any method that may achieve the result sought. The RTK antagonists may be administered, for example, orally, parenterally (intravenously or intramuscularly), topically, transdermally or by inhalation. The extracellular RTK antagonist and the intracellular RTK antagonist may be administered concomitantly or sequentially. The term mammal as used herein is intended to include, but is not limited to, humans, laboratory animals, domestic pets and farm animals. Administering a therapeutically effective amount means an amount of the compound of the present invention that, when administered to a mammal, is effective in producing the desired therapeutic effect, such as inhibiting kinase activity.

While not intending to be bound to any particular mechanism, the diseases and conditions that may be treated or prevented by the present methods include diseases and conditions associated with cellular proliferation, such as, for example, tumors, cardiovascular disease, inflammatory disease, and other proliferative diseases. Tumors that may be treated include primary tumors and metastatic tumors, as well as refractory tumors. Refractory tumors include tumors that fail to respond or are resistant to treatment with chemotherapeutic agents alone, antibodies alone, radiation alone or combinations thereof. Refractory tumors also encompass tumors that appear to be inhibited by treatment with such agents, but recur up to five years, sometimes up to ten years or longer after treatment is discontinued.

Furthermore, tumors that may be treated with the extracellular and intracellular RTK antagonists of the present invention include those that express RTKs at normal levels and are characterized by normal levels of RTK activity. The antagonists are also useful for treating tumors that overexpress RTKs, for example at levels that are at least 10, 100 or 1000 times normal levels. Such overexpression may be due to, e.g., receptor gene amplification, increased transcription or reduction in protein turnover (increased receptor stability).

Furthermore, antagonists of the present invention are useful for treating tumors that exhibit increased RTK activity due to defects in receptor signaling, for example, from mutations that result in unregulated receptor activity. Such mutant receptors may not be dependent on ligand binding for stimulation. *See, e.g.,* Pedersen et al., Ann. Oncol., 12(6):745-60 (2001). (Type III EGFR mutation ― variously named EGFRvIII, de2-7 EGFR or AEGFR ― lacks a portion of the extracellular ligand binding domain encoded by exons 2-7.); *see also* Wikstrand et al., Cancer Res., 55:3140-8 (1995).

For example, enhanced activity and overexpression of EGFR is often associated with tumor progression, and the amplification and/or overexpression of EGF receptors on tumor cell membranes has been associated with low response rates to chemotherapy and radioresistance. In another example, HER2 protein overexpression is observed in 25%-30% of primary breast cancers, which can be determined using IHC assays (e.g., HercepTest™) and gene amplification can be determined using FISH assays (e.g., Path Vysion™) of fixed tumor blocks.

Accordingly, tumors that express EGFR and are stimulated by a ligand of EGFR that can be treated using the extracellular and intracellular antagonists of the present invention include carcinomas, gliomas, sarcomas, adenocarcinomas, adenosarcomas, and adenomas. Such tumors can occur in virtually all parts of the body, including, for example, breast, heart, lung, small intestine, colon, spleen, kidney, bladder, head and neck, ovary, prostate, brain, pancreas, skin, bone, bone marrow, blood, thymus, uterus, testicles, cervix or liver. Tumors observed to overexpress EGFR that may be treated according to the present invention include, but are not limited to, colorectal and head and neck tumors, especially squamous cell carcinoma of the head and neck, brain tumors such as glioblastomas, and tumors of the lung, breast, pancreas, esophagus, bladder, kidney, ovary, cervix, and prostate. Non-limiting examples of tumors observed to have constitutively active (i.e., unregulated) receptor tyrosine kinase activity include gliomas, non-small-cell lung carcinomas, ovarian carcinomas and prostate carcinomas.

The extracellular and intracellular RTK antagonists of the present invention are also useful for treating tumors that express VEGF receptors, especially KDR. Such tumors are characteristically sensitive to VEGF present in their environment, and may further produce and be stimulated by VEGF in an autocrine stimulatory loop. The method is therefore effective for treating a solid or non-solid tumor that is not vascularized, or is not yet substantially vascularized. Examples of solid tumors that may be accordingly treated include breast carcinoma, lung carcinoma, colorectal carcinoma, pancreatic carcinoma, glioma and lymphoma. Examples of non-solid tumors include leukemia, multiple myeloma and lymphoma. Some examples of leukemias include acute myelogenous leukemia (AML), chronic myelogenous leukemia (CML), acute lymphocytic leukemia (ALL), chronic lymphocytic leukemia (CLL), erythrocytic leukemia or monocytic leukemia. Some examples of lymphomas include Hodgkin's and non-Hodgkin's lymphoma.

The extracellular and intracellular RTK antagonists of the present invention can also be used to inhibit angiogenesis. VEGFR stimulation of vascular endothelium is associated with angiogenic diseases and vascularization of tumors. Typically, vascular endothelium is stimulated in a paracrine fashion by VEGF from other sources (e.g., tumor cells). Accordingly, methods of the present invention can be effective for treating subjects with vascularized tumors or neoplasms or angiogenic diseases. Such tumors and neoplasms include, for example, malignant tumors and neoplasms, such as blastomas, carcinomas or sarcomas, and highly vascular tumors and neoplasms. Cancers that may be treated by the methods of the present invention include, for example, cancers of the brain, genitourinary tract, lymphatic system, stomach, renal, colon, larynx and lung and bone. Non-limiting examples further include epidermoid tumors, squamous tumors, such as head and neck tumors, colorectal tumors, prostate tumors, breast tumors, lung tumors, including lung adenocarcinoma and small cell and non-small cell lung tumors, pancreatic tumors, thyroid tumors, ovarian tumors, and liver tumors.

The methods of the present invention can also be used for treatment of vascularized skin cancers, including squamous cell carcinoma, basal cell carcinoma, and skin cancers that can be treated by suppressing the growth of malignant keratinocytes, such as human malignant keratinocytes. Other cancers that can be treated include Kaposi's sarcoma, CNS neoplasms (neuroblastomas, capillary hemangioblastomas, meningiomas and cerebral metastases), melanoma, gastrointestinal and renal carcinomas and sarcomas, rhabdomyosarcoma, glioblastoma, including glioblastoma multiforme, and leiomyosarcoma.

The present invention also contemplates using extracellular and intracellular RTK antagonists to treat or prevent pathologic conditions characterized by excessive angiogenesis, involving, for example, vascularization and/or inflammation, such as atherosclerosis, rheumatoid arthritis (RA), neovascular glaucoma, proliferative retinopathy including proliferative diabetic retinopathy, macular degeneration, hemangiomas, angiofibromas, and psoriasis. Other non-limiting examples of non-neoplastic angiogenic disease are retinopathy of prematurity (retrolental fibroplastic), corneal graft rejection, insulin-dependent diabetes mellitus, multiple sclerosis, myasthenia gravis, Crohn's disease, autoimmune nephritis, primary biliary cirrhosis, acute pancreatitis, allograph rejection, allergic inflammation, contact dermatitis and delayed hypersensitivity reactions, inflammatory bowel disease, septic shock, osteoporosis, osteoarthritis, cognition defects induced by neuronal inflammation, Osler-Weber syndrome, restinosis, and fungal, parasitic and viral infections, including cytomegaloviral infections. The foregoing diseases and conditions are only illustrative and the methods of the present invention are not limited to treating only the exemplified diseases and conditions but rather any disease or condition that may be treated by regulation of kinases.

Moreover, included within the scope of the present invention is use of the present inventive compounds *in vivo* and *in vitro* for investigative or diagnostic methods, which are well known in the art.

Another aspect of the present invention relates to pharmaceutical compositions containing the antagonists of the present invention or a pharmaceutically acceptable salt, hydrate or pro-drug thereof, in combination with a pharmaceutically acceptable carrier. Such compositions may be separate compositions of the extracellular RTK antagonist and the intracellular RTK antagonist or a single composition containing both the extracellular and intracellular RTK antagonists.

The compositions of the present invention may be in solid or liquid form, in solution or in suspension. Routes of administration include, for example, oral, parenteral (intravenous, intraperitoneal, subcutaneous, or intramuscular), topical, transdermal and by inhalation.

For oral administration, the RTK antagonists may be administered, for example, in liquid form with an inert diluent or assimilable carrier, or incorporated into a solid dosage form. Examples of oral liquid and solid dosage forms include, for example, solutions, suspensions, syrups, emulsions, tablets, lozenges, capsules (including soft gelatin capsules), and the like. Oral dosage forms may be formulated as sustained release products using, for example, a coating to delay disintegration or to control diffusion of the active compound. Where necessary, the compositions may also include a solubilizing agent.

Examples of injectable dosage forms include sterile injectable liquids, including, for example, solutions, emulsions and suspensions. Injectable dosage forms further include solids such as sterile powders that are reconstituted, dissolved or suspended in a liquid prior to injection. Sterile injectable solutions are prepared by incorporating the RTK antagonists in the required amount in the appropriate solvent with various of the other ingredients enumerated above, as required, followed by filtered sterilization. Carriers typically include, for example, sterile water, saline, injectable organic esters, peanut oil, vegetable oil, and the like. Buffering agents, preservatives, and the like can be included in the administerable forms. Sterile formulations can be prepared by heating, irradiation, microfiltration, and/or by addition of various antibacterial and antifungal agents, such as, for example, parabens, chlorobutanol, phenol, sorbic acid, thimerosal, and the like.

For topical administration, RTK antagonists of the present invention can be administered, for example, in the form of gels, creams, or ointments, or paints. Typical carriers for such application include hydrophobic or hydrophilic bases, oleaginous or alcoholic liquids, and dry powders. RTK antagonists may be also incorporated in a gel or matrix base for application in a patch, optionally providing for controlled release of compound through a transdermal barrier. RTK antagonists can also be formulated by known methods for rectal administration.

For administration by inhalation, RTK antagonists of the present invention may be dissolved or suspended in, or adsorbed onto, a suitable carrier for use in a nebulizer, aerosol, or dry powder inhaler.

Suitable dosages can be determined by a physician or qualified medical professional, and depend on factors such as the nature of the illness being treated, the route of administration, the duration of the treatment, and the condition of the patient. The RTK antagonists of the present invention may be administered as frequently as necessary in order to obtain the desired therapeutic effect. Frequency of administration will depend, for example, on the nature of the dosage form used and the disease being treated. An exemplary dosage of current extracellular EGFR antagonists is 400 mg/m² loading and 250 mg/m² weekly infusion (cetuximab); 1.5 mg/kg weekly infusion (ABX-EGF); and a 4 mg/kg loading dose administered as a 90-minute infusion and a maintenance dose of 2 mg/kg as a 30 minute infusion (trastuzumab). An exemplary dosage of current intracellular EGFR antagonists is 250 mg/day oral administration (Iressa); 150 mg/day oral administration (Tarceva); and 560 mg/weekly oral administration (CI-1033).

Because the present invention provides a treatment that may function by two different, independent mechanisms, such a treatment provides an enhanced or synergistic effect on tumor inhibition as compared to administration of either solely an extracellular antagonist or an intracellular antagonist. Furthermore, because the present invention provides treatment with an extracellular RTK antagonist and an intracellular RTK antagonist, the therapeutically effective dose may be lower than the therapeutically effective dose of either an extracellular RTK antagonist alone or an intracellular RTK antagonist alone.

Unlike current treatment that require continuous dosing in order to suppress tumor growth, the combination therapy of the present invention permits intermittent dosing of the extracellular and intracellular RTK antagonists to suppress tumor growth. For example, the two treatments can be administered simultaneously. Alternatively, the two treatments can be administered sequentially. In addition, the two treatments can be administered cyclically. Thus, the two antagonists may be administered concurrently for a period of time, and then one or the other administered alone. Of course, any combination or order of administration may be used.

In another aspect of the present invention, the extracellular and intracellular RTK antagonists of the present invention are formulated for use in conjunction with other therapeutically active compounds or are administered in connection with the application of therapeutic techniques. Any conventional therapy known in the art can be used in combination with the present inventive methods.

For example, the extracellular and intracellular RTK antagonists can be administered in combination with one or more other antineoplastic agents. *See, e.g.,* U.S. Patent No. 6,217,866 (Schlessinger et al.*)* (Anti-EGFR antibodies in combination with antineoplastic agents); U.S. Application No. 09/312,286 (Waksal et al.) (Anti-EGFR antibodies in combination with radiation). Any suitable antineoplastic agent can be used, such as a chemotherapeutic agent or radiation. Examples of chemotherapeutic agents include, but are not limited to, cisplatin, doxorubicin, paclitaxel, irinotecan (CPT-11), topotecan, and oxaliplatin, or a combination thereof. When the antineoplastic agent is radiation, the source of the radiation can be either external (external beam radiation therapy ― EBRT) or internal (brachytherapy ― BT) to the patient being treated. The dose of antineoplastic agent administered depends on numerous factors, including, for example, the type of agent, the type and severity tumor being treated and the route of administration of the agent. It should be emphasized, however, that the present invention is not limited to any particular dose.

In addition, the extracellular and intracellular RTK antagonist can be administered in combination with one or more suitable adjuvants, such as, for example, cytokines (IL-10 and IL-13, for example) or other immune stimulators. *See, e.g.,* Larrivée et al., Int'l J. Mol. Med., 5: 447-56 (2000).

The foregoing description has been set forth merely to illustrate the invention and is not intended to be limiting. Modifications of the disclosed embodiments incorporating the spirit and substance of the invention may occur to persons skilled in the art and such modifications are within the scope of the present invention. Furthermore, all references cited herein are incorporated by reference in their entirety.

### Embodiments of the invention are:

1. A method of inhibiting a receptor tyrosine kinase (RTK) in a mammal comprising administering an extracellular RTK antagonist and an intracellular RTK antagonists to the mammal.
2. The method of embodiment 1, wherein the method is used to treat a tumor growth or angiogenesis in the mammal.
3. The method of embodiment 1 or 2, wherein the RTK is Epidermal Growth Factor Receptor (EGFR).
4. The method of embodiment 3, wherein the extracellular RTK antagonist is cetuximab, ABX-EGF, EMD 72000, h-R3, or Y10.
5. The method of embodiment 3, wherein the intracellular RTK antagonist is ZD1939 or OSI-774.
6. The method of embodiment 1 or 2, wherein the RTK is HER2 receptor.
7. The method of embodiment 6, wherein the extracellular RTK antagonist is trastuzumab.
8. The method of embodiment 1 or 2, wherein the RTK is Vascular Endothelial Growth Factor Receptor (VEGFR).
9. The method of embodiment 8, wherein the extracellular RTK antagonist is bevacizumab.
10. The method of embodiment 1 or 2, wherein the intracellular RTK antagonist inhibits ras protein or a ras-raf modulator.
11. The method of any one of embodiments 1-10, wherein the method further comprises administrating an antineoplastic agent.
12. A pharmaceutical composition comprising an extracellular RTK antagonist and an intracellular RTK antagonist.
13. The pharmaceutical composition of embodiment 12, wherein the RTK is Epidermal Growth Factor Receptor (EGFR).
14. The pharmaceutical composition of embodiment 13, wherein the extracellular RTK antagonist is cetuximab, ABX-EGF, EMD 72000, h-R3, or Y10.
15. The pharmaceutical composition of embodiment 13 or 14, wherein the intracellular RTK antagonist is ZD1939 or OSI-774.
16. The pharmaceutical composition of any one of embodiments 12-15, wherein the RTK is HER2 receptor.
17. The pharmaceutical composition of embodiment 16, wherein the extracellular RTK antagonist is trastuzumab.
18. The pharmaceutical composition of embodiment 12, wherein the RTK is Vascular Endothelial Growth Factor Receptor (VEGFR).
19. The pharmaceutical composition of embodiment 18, wherein the extracellular RTK antagonist is bevacizumab.
20. The pharmaceutical composition of embodiment 12, wherein the intracellular RTK antagonist inhibits ras protein or a ras-raf modulator.
21. The pharmaceutical composition of any one of embodiments 12-20, wherein the pharmaceutical composition further comprises an antineoplastic agent.

## Claims

1. An extracellular receptor tyrosine kinase (RTK) antagonist and an intracellular RTK antagonist for use in inhibiting tumor growth or angiogenesis in a mammal.

2. The extracellular RTK antagonist and intracellular RTK antagonist of claim 1, wherein the extracellular RTK antagonist and the intracellular RTK antagonist inhibit the same RTK.

3. The extracellular RTK antagonist and intracellular RTK antagonist of claim 1, wherein the extracellular RTK antagonist inhibits a first RTK and the intracellular RTK antagonist inhibits a second RTK.

4. The extracellular RTK antagonist and intracellular RTK antagonist of claim 1 or 2, wherein the RTK is
(a) a vascular endothelial growth factor receptor (VEGFR);
(b) a platelet-derived growth factor receptor (PDGFR);
(c) a fibroblast growth factor receptor (FGFR);
(d) a nerve growth factor receptor (NGFR);
(e) an epidermal growth factor receptor; or
(f) an insulin-like growth factor receptor (IGF-IR) or insulin receptor-related receptor (IRR).

5. The extracellular RTK antagonist and intracellular RTK antagonist of claim 3, wherein the first RTK is
(a) a vascular endothelial growth factor receptor (VEGFR);
(b) a platelet-derived growth factor receptor (PDGFR);
(c) a fibroblast growth factor receptor (FGFR);
(d) a nerve growth factor receptor (NGFR);
(e) an epidermal growth factor receptor; or
(f) an insulin-like growth factor receptor (IGF-IR) or insulin receptor-related receptor (IRR).

6. The extracellular RTK antagonist and intracellular RTK antagonist of any one of claims 1 to 3, wherein the intracellular RTK antagonist is ZD-6474, ZD1839, OSI-774, or a ras-raf modulator.

7. The extracellular RTK antagonist and intracellular RTK antagonist of any one of claims 1 to 3, wherein the extracellular RTK antagonist is cetuximab, ABX-EGF, EMD 72000, h-R3, Y10, trastuzumab, or bevacizumab.

8. The extracellular RTK antagonist and intracellular RTK antagonist of any one of claims 3 to 6, wherein the second RTK is a VEGFR, a PDGFR, a FGFR, a NGFR, an EGFR, IGF-IR, or IRR.

9. The extracellular RTK antagonist and intracellular RTK antagonist of claim 4, 5 or 8, wherein the VEGFR is VEGFR-1, VEGFR-2, VEGFR-3, neuropilin-1, or neuropilin-2.

10. The extracellular RTK antagonist and intracellular RTK antagonist of any one of claims 1 to 9, wherein the use further comprises administering an antineoplastic agent.

11. A combination of an extracellular RTK antagonist and an intracellular RTK antagonist for inhibiting tumor growth or angiogenesis in a mammal.

12. The combination of claim 11, wherein the extracellular RTK antagonist and the intracellular RTK antagonist inhibit the same RTK.

13. The combination of claim 11, wherein the extracellular RTK antagonist inhibits a first RTK and the intracellular RTK antagonist inhibits a second RTK.

14. The combination of claim 11 or 12, wherein the RTK is
(a) a vascular endothelial growth factor receptor (VEGFR);
(b) a platelet-derived growth factor receptor (PDGFR);
(c) a fibroblast growth factor receptor (FGFR);
(d) a nerve growth factor receptor (NGFR);
(e) an epidermal growth factor receptor; or
(f) an insulin-like growth factor receptor (IGF-IR) or insulin receptor-related receptor (IRR).

15. The combination of claim 13, wherein the first RTK is
(a) a vascular endothelial growth factor receptor (VEGFR);
(b) a platelet-derived growth factor receptor (PDGFR);
(c) a fibroblast growth factor receptor (FGFR);
(d) a nerve growth factor receptor (NGFR);
(e) an epidermal growth factor receptor; or
(f) an insulin-like growth factor receptor (IGF-IR) or insulin receptor-related receptor (IRR).

16. The combination of any one of claims 11 to 13, wherein the intracellular RTK antagonist is ZD-6474, ZD1839, OSI-774, or a ras-raf modulator.

17. The combination of any one of claims 13 to 16, wherein the second RTK is a VEGFR, a PDGFR, a FGFR, a NGFR, a EGFR, IGF-IR, or IRR.

18. The combination of any one of claims 11 to 13, wherein the extracellular RTK antagonist is cetuximab, ABX-EGF, EMD 72000, h-R3, Y10, trastuzumab, or bevacizumab.

19. The combination of claim 14, 15 or 17, wherein the VEGFR is VEGFR-1, VEGFR-2, VEGFR-3, neuropilin-1, or neuropilin-2.

20. The combination of any one of claims claim 11 to 19, further comprising an antineoplastic agent.
